# DEMANDE DE BREVET EUROPEEN

(11) **EP 0 638 641 A1**
(43) Date de publication de la demande: **15.02.1995**
(21) Numéro de dépôt: 94401588.2
(22) Date de dépôt: 08.07.1994
(51) Int. Cl.: C12M 3/00, C12M 1/00

(54) **Bioréacteur miniature à renouvellement continu de milieu nutritif**

(30) Priorité: 12.07.1993 FR 9308561
(71) Demandeur: CARRAR, F-78104 Saint Germain en Laye Cédex (FR)
(72) Inventeur: Devidts, Gilles, F-78100 Saint Germain en Laye (FR); Dominici, Bernard, F-75011 Paris (FR); Bouet, Thierry, F-92420 Vaucresson (FR); Germain, Jean-Claude, F-91190 Gif sur Yvette (FR)
(74) Mandataire: Loisel, Bertrand

(57) **Abrégé**

Le réacteur permet de renouveler de façon continue le milieu nutritif dans la chambre de culture (1). La circulation des fluides est assurée en circuit fermé par une pompe (2) intégrée, agissant sur un circuit hydraulique (6b), poussant le milieu nutritif neuf du réservoir (4) dans la chambre de culture (1), puis dans le réservoir de milieu usé (3) séparé du milieu neuf (4) par une paroi souple (5).

L'expérimentateur peut observer les cellules en cours de culture sans ouvrir le réacteur ni interrompre le renouvellement.

## Description

La présente invention se rapporte à un dispositif de culture cellulaire in vitro.

Les documents WO 86/07604 et WO 86/07605 décrivent des bioréacteurs conventionnels conçus pour des cultures de masse. Il y a deux types de conception de la chambre de culture :
- cuve en verre avec agitation mécanique pour cellules en suspension ou adhérentes sur microsphères (volume de la chambre de culture supérieur à 1 litre). Les échanges de milieu se font par convection,
- module de fibres creuses dans lesquelles passe le milieu de culture, les cellules se développant à l'extérieur (volume utile de 100 millilitres).

Dans les deux cas, l'observation des cellules est impossible en cours de culture. Ces réacteurs ne sont pas destinés à des expériences de recherche ou de criblage sur des petites quantités. Les applications de ces réacteurs sont donc sévèrement limitées, principalement parce qu'ils ne sont pas aisément miniaturisables. Or les expériences de culture cellulaire in vitro concernent souvent des quantités limitées de cellules et de milieu nutritif.

Un but de l'invention est de proposer un bioréacteur qui soit aisément miniaturisable et qui puisse fonctionner avec un minimum d'interventions de l'utilisateur. L'invention vise encore à éviter la contamination du milieu extérieur à la chambre de culture et à permettre l'observation de la réaction sans avoir à ouvrir la chambre de culture.

L'invention propose ainsi un bioréacteur comprenant au moins une chambre de culture, un réservoir de milieu neuf, un réservoir de milieu usé, et un circuit comportant une pompe pour assurer un transfert de fluide du réservoir de milieu neuf vers la chambre de culture et de la chambre de culture vers le réservoir de milieu usé, caractérisé en ce que les réservoirs sont logés dans un compartiment commun, de part et d'autre d'une séparation souple

Le réacteur selon l'invention permet d'éviter les opérations manuelles, habituelles en culture cellulaire, de changement de milieu, recueil des effluents, fixation chimique des cellules. Ainsi, selon la vitesse de croissance des types cellulaires cultivés, l'autonomie du réacteur sans ouverture ni manipulation des cellules, peut atteindre plusieurs dizaines de jours.

De plus, le réacteur permet aisément de récupérer le milieu usé, pour analyse. Ce réacteur est adapté aux cultures anaérobies, ou aérobies sous atmosphère contrôlée, en le plaçant dans un incubateur.

Le bioréacteur selon l'invention permet de réaliser des cultures dans de petits volumes et plusieurs cultures simultanément.

L'encombrement du réacteur permet alors de le placer (le cas échéant en plusieurs exemplaires) dans un incubateur de laboratoire conventionnel ainsi que dans tout autre environnement, de température, d'éclairement, d'atmosphère...

La chambre, dans laquelle le milieu de culture se renouvelle en continu par diffusion, convient pour la culture de cellules en suspension ou adhérentes (en ajoutant un ou plusieurs supports) avec des petites quantités de cellules. La chambre de culture présente avantageusement une zone transparente pour permettre l'observation optique de son contenu, c'est-à-dire des cellules en croissance.

Ce réacteur trouve des applications dans les laboratoires effectuant des cultures de cellules de petit volume, lors d'expériences à procédures systématiques pharmacologie, bactériologie, toxicologie, dermatologie in vitro, essais de mutagénicité, cytotoxicité ...

L'invention est décrite plus en détail en regard des dessins donnés en annexe à titre d'exemple non limitatif, et dans lesquels :
- la figure 1 représente un bioréacteur selon l'invention en coupe selon le plan axial, comportant un seul compartiment muni de réservoirs de milieux neuf et usé ;
- la figure 2 est une vue en coupe dans un plan radial A-A indiqué à la figure 3 d'une pompe péristaltique utilisable dans le bioréacteur de la figure 1 ;
- la figure 3 est une vue en coupe de cette pompe dans le plan axial B-B indiqué à la figure 2 ;
- la figure 4 est une vue en coupe suivant le plan C-C indiqué à la figure 5 de la bague extérieure d'un palier faisant partie de la pompe des figures 2 et 3 ;
- la figure 5 est une vue de dessus de cette bague extérieure ; et
- la figure 6 illustre un mode particulier de réalisation du réacteur selon l'invention, en coupe selon un plan axial, comportant trois chambres de culture.

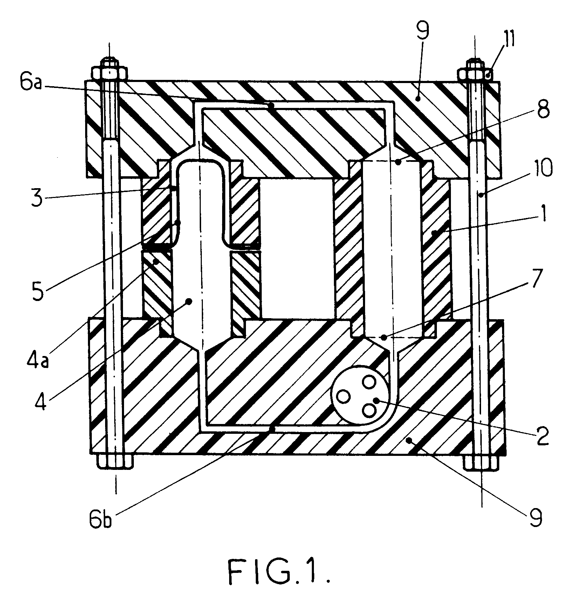

En référence à la figure 1, un bioréacteur selon l'invention comprend une chambre de culture 1 et un compartiment 4a destiné à former deux réservoirs 3, 4. La chambre 1 et le compartiment 4a sont des éléments tubulaires réalisés dans un matériau cyto-compatible et stérilisable, tel que du polycarbonate. Ces éléments tubulaires 1, 4a ont la même longueur et sont montés entre deux pièces formant support 9 assemblées par exemple au moyen de vis 10 et d'écrous 11.

Dans le compartiment 4a, le réservoir de milieu neuf 4 et le réservoir de milieu usé 3 sont disposés de part et d'autre d'une séparation souple 5 imperméable aux fluides.

Un circuit de transfert de fluides est constitué entre la chambre 1 et les réservoirs 3, 4. Ce circuit comprend une partie 6a formée dans une pièce formant support 9, pour assurer un transfert de fluides de la chambre de culture 1 vers le réservoir de milieu usé 3, et une partie 6b formée dans l'autre pièce formant support 9 pour assurer un transfert de fluides du réservoir de milieu neuf 4 vers la chambre de culture 1. Cette seconde partie de circuit 6b comporte une pompe 2 permettant de commander les transferts de fluides. Ces transferts s'effectuent à volume global constant : le transfert d'un volume de milieu de culture neuf du réservoir 4 vers la chambre 1 s'accompagne du transfert d'un volume équivalent de milieu de culture usé de la chambre 1 vers le réservoir 3. Ces transferts entraînent un déplacement de la séparation souple 5 dans le compartiment 4a.

Deux membranes microporeuses 7, 8 sont placées respectivement à l'entrée et à la sortie de la chambre de culture 1. La taille des pores de ces membranes peut par exemple être de 0,45 µm.

Dans un bioréacteur miniature, la chambre de culture 1 et le compartiment 4a auront typiquement un volume compris entre 0,5 ml et 15 ml.

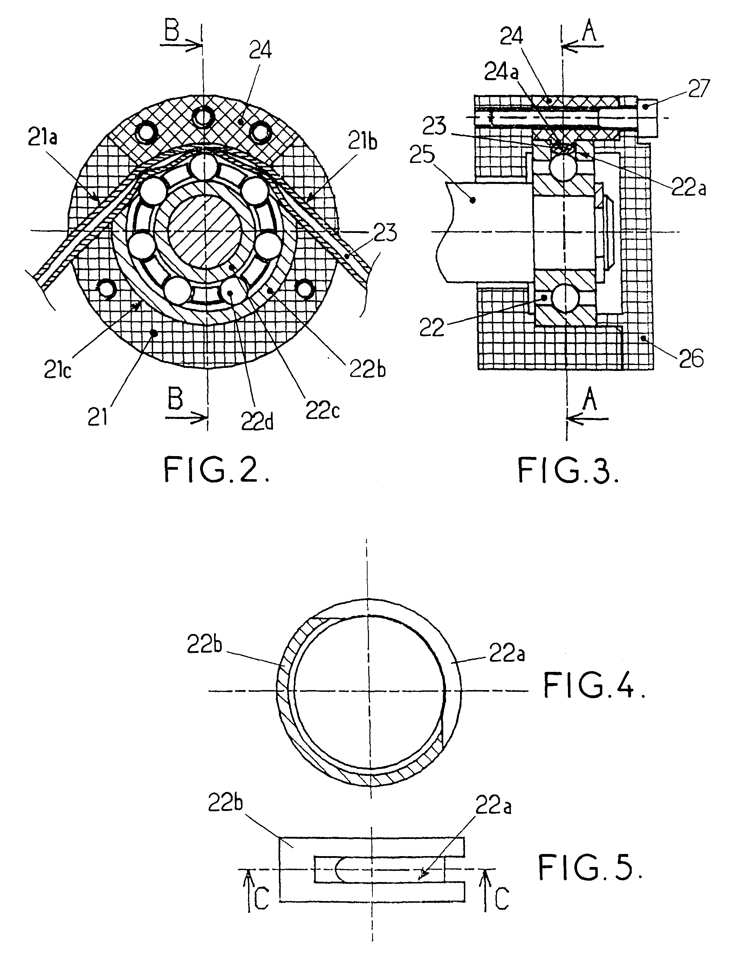

Les pièces-support 9 peuvent être réalisées dans le même matériau cyto-compatible que la chambre de culture 1. L'une des pièces 9 comporte une cavité pour recevoir la pompe 2, qui peut être une pompe péristaltique telle que celle représentée aux figures 2 à 5. Cette pompe comprend un boîtier 21 fixé dans la cavité de la pièce 9 et comportant un alésage 21c dans lequel est fixé un palier 22 de type roulement, par exemple un roulement à billes. La bague extérieure 22b du palier 22 constitue le stator de la pompe, tandis que la bague intérieure 22c en constitue le rotor. Une fente 22a est usinée dans la bague extérieure 22b du palier, dans le plan médian du palier perpendiculaire à son axe de rotation (figures 4 et 5). Cette fente reçoit un tronçon d'un tube déformable 23 appartenant à la partie de circuit 6b et en définit la zone de laminage. La largeur de la fente 22a est de préférence égale à la largeur du tube 23 lorsqu'il est écrasé, comme le montre la figure 3. Le boîtier 21 est muni de deux orifices 21a et 21b pour le passage du tube 23, qui débouchent dans l'alésage 21c du boîtier, tangentiellement à celui-ci en face des deux extrémités de la fente et dans la continuité de celle-ci, et d'une pièce amovible 24 constituant le secteur d'arc de l'alésage 21c situé en vis-à-vis de la fente 22a, et jouant rôle de matrice pour l'écrasement du tube 23 (figures 2 et 3). La bague intérieure 22c du palier, qui constitue le rotor pour les corps roulants 22d, est fixée sur un arbre d'entraînement 25 relié à un moteur électrique non représenté. Un capot 26 est fixé au moyen de vis 27 sur le boîtier 21, à son extrémité opposée à l'arbre 25, afin de maintenir en place la bague extérieure 22b du palier et la pièce amovible 24. Comme le montre la figure 3, la pièce amovible 24 est munie, sur sa face intérieure, d'une bande en surépaisseur 24a qui s'insère dans la fente 22a afin d'assurer la force d'écrasement désirée sur le tube 23. Un chanfrein est pratiqué à chaque extrémité de cette bande 24a pour éviter les chocs au passage des corps roulants 22d.

Cette pompe 2, qui fait l'objet d'une demande de brevet français parallèle N° 93 08562, convient particulièrement pour réaliser un bioréacteur miniature. A titre d'exemple, l'encombrement de la pompe peut être celui d'une pastille de 12 mm de diamètre sur une hauteur de 5 mm, pour un débit continu de 0,1 µl par heure à une vitesse de rotation de 1 tour par heure.

Pour préparer une expérience de culture cellulaire, on dispose les cellules à cultiver dans la chambre 1, et on remplit le réservoir de milieu neuf 4, la séparation souple 5 prenant alors la position indiquée sur la figure 1. L'ensemble du réacteur peut être placé dans un incubateur pour réaliser les conditions physiques et chimiques désirées pour la réaction.

La chambre de culture 1 comporte avantageusement au moins une partie réalisée dans un matériau imperméable aux liquides mais perméable aux gaz (O₂ et CO₂ notamment), par exemple une résine silicone telle que celle commercialisée sous l'appellation "Silastic Q7-2213, medical grade" par la firme Dow Corning. Cet agencement permet d'effectuer des cultures aérobies.

Pour permettre l'observation en continu de la réaction, la chambre de culture 1 présente une zone transparente qui peut être obtenue en polissant le polycarbonate. La zone transparente est de préférence plane.

La vitesse de renouvellement du milieu de culture peut être réglée en commandant de façon appropriée la pompe 2, pour obtenir le taux de croissance désiré des cellules cultivées. L'ensemble de la réaction peut durer entre quelques heures et quelques dizaines de jours.

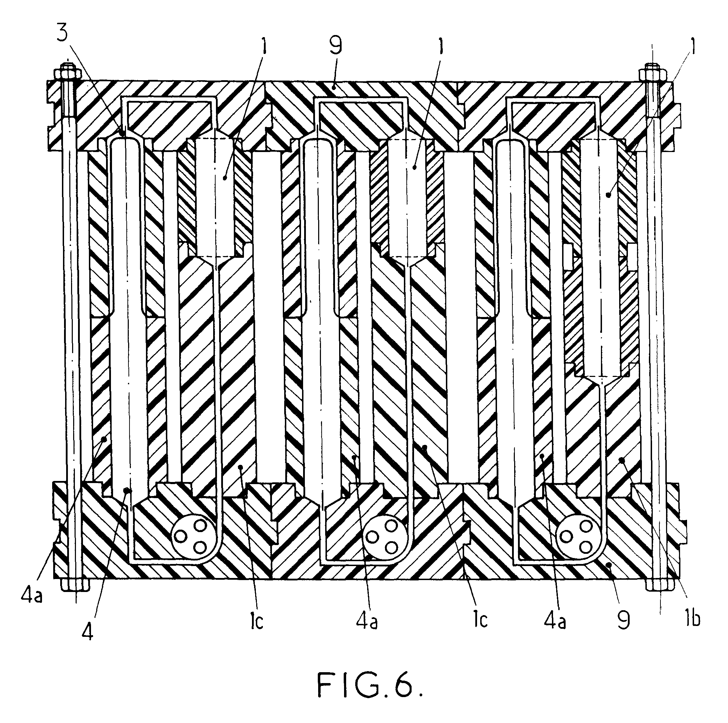

Selon un mode de montage particulier, illustré sur la figure 6, plusieurs chambres de culture 1 et plusieurs compartiments 4a sont réunis dans un même ensemble. Les pièces supports 9, adaptées pour recevoir plusieurs chambres 1 et plusieurs compartiments 4a, peuvent être réalisées soit d'un seul tenant soit, comme représenté, par assemblage de plusieurs éléments.

Sur la figure 6, on voit qu'il est possible d'utiliser pour une chambre de culture 1 un ou plusieurs éléments tubulaires plus courts que celui utilisé pour le compartiment associé 4a. Dans ce cas, on interpose un élément tubulaire de raccordement 1b, 1c entre une pièce-support 9 et la chambre, cet élément ayant une section intérieure plus faible que celle de la chambre et formant une partie du circuit de transfert de fluide. Un agencement analogue est possible lorsque la chambre 1 est plus longue que le compartiment 4a.

Le réacteur de la figure 6 a un caractère modulaire. On peut aussi, en utilisant des pièces-supports appropriées, mettre une chambre de culture 1 en communication avec plusieurs compartiments à réservoirs.

## Revendications

1. Bioréacteur comprenant au moins une chambre de culture (1), un réservoir de milieu neuf (4), un réservoir de milieu usé (3), et un circuit (6a,6b) comportant une pompe (2) pour assurer un transfert de fluide du réservoir de milieu neuf vers la chambre de culture et de la chambre de culture vers le réservoir de milieu usé, caractérisé en ce que les réservoirs (4,3) sont logés dans un compartiment commun (4a), de part et d'autre d'une séparation souple (5).

2. Bioréacteur selon la revendication 1, caractérisé en ce que deux membranes microporeuses (7,8) sont placées respectivement à l'entrée et à la sortie de la chambre de culture (1).

3. Bioréacteur selon la revendication 1 ou 2, caractérisé en ce que la chambre de culture (1) est au moins partiellement réalisée dans un matériau imperméable aux liquides, mais perméable aux gaz.

4. Bioréacteur selon l'une quelconque des revendications 1 à 3, caractérisé par le fait que la chambre de culture (1) présente au moins une zone transparente.

5. Bioréacteur selon l'une quelconque des revendications 1 à 4, caractérisé en ce que la chambre de culture (1) et le compartiment (4a) où sont logés les réservoirs (3,4) sont constitués par des éléments tubulaires montés entre des pièces formant support (9) incorporant au moins une partie du circuit de fluide (6a,6b).

6. Bioréacteur selon la revendication 5, caractérisé en ce que les pièces formant support (9) sont adaptées pour recevoir plusieurs chambres de culture (1) et plusieurs compartiments à réservoirs (4a).

7. Bioréacteur selon la revendication 5 ou 6, caractérisé en ce qu'il comprend au moins un élément tubulaire de raccordement (1b,1c) interposé entre une pièce formant support (9) et la chambre de culture (1) ou le compartiment à réservoirs (4a) pour compenser une différence de longueur entre les éléments tubulaires formant la chambre de culture et le compartiment .

8. Bioréacteur selon l'une quelconque des revendications 1 à 7, caractérisé en ce que le compartiment à réservoirs (4a) et la chambre de culture (1) ont un volume compris entre 0,5 ml et 15 ml.

9. Bioréacteur selon l'une quelconque des revendications 1 à 8, caractérisé en ce que la pompe (2) est une pompe péristaltique.
